# EUROPEAN PATENT APPLICATION

(11) **EP 4 212 024 A1**
(43) Date of publication of application: **19.07.2023**
(21) Application number: 21866836.6
(22) Date of filing: 09.09.2021
(51) Int. Cl.: A23L 11/45

(54) **TOFU PRODUCTION METHOD**

(30) Priority: 10.09.2020 JP 2020152013
(71) Applicant: Amano Enzyme Inc., Nagoya-shi Aichi 460-8630 (JP)
(72) Inventor: SAKAI, Kiyota, Kakamigahara-shi, Gifu 509-0109 (JP)
(74) Representative: Müller-Boré & Partner Patentanwälte PartG mbB
(86) International application number: PCT/JP2021/033198
(87) International publication number: WO 2022/054879

(57) **Abstract**

The purpose of the present invention is to provide a novel feature by which the strength of tofu can be increased with a method for producing tofu using an enzyme. Tofu obtained by a tofu production method that includes a crosslinking step in which laccase is acted on soy milk exhibits increased strength, and when the tofu production method thereof further includes a boiling step after the crosslinking step, the strength of the obtained tofu is dramatically improved.

## Description

### TECHNICAL FIELD

The present invention relates to a tofu production method. More specifically, the present invention relates to a method for producing tofu with enhanced strength.

### BACKGROUND ART

Unlike animal protein sources, plant protein sources have health advantageous characteristics such as low lipid and inclusion of fiber, and have been increasingly attracting attention with increasing health consciousness.

Among them, tofu is regarded as a typical health food in which soybean protein is most easily eaten. While tofu is a traditional food, in recent years, there is a growing need for modification of taste and/or texture of the tofu. One of such modifications is enhancement of the gel strength of tofu intended to improve the sense of fillingness and/or enable adaptability to various cooking methods.

For example, Patent Document 1 describes that sufficient hardness can be obtained by adding transglutaminase even when tofu is produced using soybeans of a variety forming soft tofu as a raw material. Patent Document 2 describes that the strength of tofu is improved by adding glucose oxidase and an iron-containing yeast to soy milk.

### PRIOR ART DOCUMENTS

### PATENT DOCUMENTS

Patent Document 1: Japanese Patent Laid-open Publication No. 2007-312723
Patent Document 2: Japanese Patent Laid-open Publication No. 2015-180197

### SUMMARY OF THE INVENTION

### PROBLEMS TO BE SOLVED BY THE INVENTION

As a method for increasing the strength of tofu, a method using a predetermined enzyme as described above is known, but further options for an enzyme to be used are desired so as to be able to cope with the increasing need for modification required for tofu.

Therefore, an object of the present invention is to a novel technique capable of increasing the strength (compressive strength) of tofu in a tofu production method using an enzyme.

### MEANS FOR SOLVING THE PROBLEM

The present inventor has found that the strength of tofu to be obtained is enhanced by causing a laccase to act on soy milk. The present inventor has also found that the strength of tofu is further dramatically enhanced when boiling sterilization is combined. That is, the present invention provides inventions of the following aspects.

Item 1. A tofu production method including a crosslinking step of causing a laccase to act on soy milk.

Item 2. The tofu production method described in item 1, further including a boiling step after the crosslinking step.

Item 3. The tofu production method described in item 1 or 2, further including a coagulating step of causing a coagulant to act.

Item 4. The tofu production method described in any one of items 1 to 3, in which the laccase is used in an amount of 5 U or more per 1 g of soybean protein.

Item 5. The tofu production method described in any one of items 1 to 4, in which the laccase is used in an amount of 30 U or more per 1 g of soybean protein.

Item 6. The tofu production method described in any one of items 1 to 5, in which the laccase is derived from Trametes hirsuta.

Item 7. The tofu production method described in any one of items 1 to 6, in which the crosslinking step is performed at 50 to 70°C.

Item 8. The tofu production method described in any one of items 3 to 7, in which the coagulant is used at a concentration of 0.1 wt% or more.

Item 9. The tofu production method described in any one of items 3 to 8, in which the coagulant is used at a concentration of 0.5 wt% or more.

Item 10. The tofu production method described in any one of items 3 to 9, in which the coagulant is magnesium chloride.

### ADVANTAGES OF THE INVENTION

According to the present invention, there is provided a novel technique capable of increasing the strength of tofu in a tofu production method using an enzyme.

### BRIEF DESCRIPTION OF THE DRAWINGS

Fig. 1 shows the relationship between the presence or absence of a laccase treatment and a difference in reaction time of a soy milk composition in production of tofu, and the strength of tofu, as obtained in Test Example 1.
Fig. 2 shows the relationship between a difference in use amount of a laccase in production of tofu and the strength of tofu, as obtained in Test Example 2.
Fig. 3 shows the relationship between the presence or absence of a laccase treatment and a difference in use amount of a coagulant in production of tofu, and the strength of tofu, as obtained in Test Example 3.
Fig. 4 shows the relationship between the presence or absence of a laccase treatment and the presence or absence of a boiling step in production of tofu, and the strength of tofu, as obtained in Test Example 4.
Fig. 5 shows the relationship between the presence or absence of a laccase treatment and a difference in reaction time of a soy milk composition in production of tofu in which a boiling step is performed, and the strength of tofu, as obtained in Test Example 5.
Fig. 6A shows the relationship between a difference in use amount of a laccase in production of tofu in which a boiling step is performed and the strength of tofu, as obtained in Test Example 6.
Fig. 6B shows the relationship between a difference in use amount of a laccase in production of tofu in which a boiling step is performed and the relative strength of tofu, as obtained in Test Example 6.
Fig. 7A shows the relationship between a difference in use amount of a coagulant in production of tofu in which a boiling step is performed without performing a laccase treatment and the strength of tofu, as obtained in Test Example 7.
Fig. 7B shows the relationship between a difference in use amount of a coagulant in production of tofu in which a laccase treatment and a boiling step are performed and the strength of tofu, as obtained in Test Example 7.
Fig. 7C shows the relationship between a difference in use amount of a coagulant in production of tofu in which a boiling step is performed without performing a laccase treatment and the relative strength of tofu, as obtained in Test Example 7.
Fig. 7D shows the relationship between a difference in use amount of a coagulant in production of tofu in which a laccase treatment and a boiling step are performed and the relative strength of tofu, as obtained in Test Example 7.

### EMBODIMENTS OF THE INVENTION

A tofu production method of the present invention includes a crosslinking step of causing a laccase to act on soy milk. Hereinafter, the tofu production method of the present invention will be described in detail.

The soy milk used in the present invention is not particularly limited, and for example, squeeze of water-absorbed soybeans, commercially available soy milk for tofu, or the like can be used. The soybean protein amount in the soy milk is not particularly limited, and is, for example, 0.5 g/100 mL or more, 1 g/100 mL or more, or 2 g/100 mL or more, 3 g/100 mL or more, preferably 3.8 g/100 mL or more, more preferably 4.2 g/100 mL or more, further preferably 4.8 g/100 mL or more, and particularly preferably 5 g/100 mL or more. The upper limit of the soybean protein amount range in the soy milk is not particularly limited, and is, for example, 15 g/100 mL or less, 10 g/100 mL or less, or 8 g/100 mL or less. The soybean solid content of the soy milk is not particularly limited, and is, for example, 2 wt% or more, 4 wt% or more, or 6 wt% or more, preferably 8 wt% or more, more preferably 9 wt% or more, further preferably 9.5 wt% or more, and particularly preferably 10 wt% or more. The upper limit of the soybean solid content of the soy milk is not particularly limited, and is, for example, 30 wt% or less, 25 wt% or less, 20 wt% or less, 18 wt% or less, or 16 wt% or less.

The soy milk used in the present invention may contain a quality improving agent such as an emulsifier (such as sucrose fatty acid ester, phospholipid, monoglyceric fatty acid ester, organic acid monoglyceric fatty acid ester, sorbitan fatty acid ester, polyoxyethylene sorbitan fatty acid ester, polyglycerin fatty acid ester, or propylene glycol fatty acid ester) and a pH adjusting agent (such as sodium carbonate, sodium hydrogen carbonate, or calcium carbonate), a coloring agent, a flavor, a seasoning, and the like as long as the effect of the present invention is not impaired.

The laccase used in the present invention is an enzyme having phenol oxidase activity (EC1.10.3.2). Specific examples of the laccase include laccases derived from microorganisms such as fungi and bacteria, and more specific examples thereof include laccases derived from the genera Aspergillus, Neurospora, Podospora, Botrytis, Collybia, Fomes, Lentinus, Pleurotus, Pycnoporus, Pyricularia, Trametes, Rhizoctonia, Rigidoporus, Coprinus, Psatyrella, Myceliophtera, Schtalidium, Polyporus, Phlebia, Coriolus, and the like.

These laccases may be used singly or in combination of a plurality of kinds thereof. Among these laccases, a laccase derived from the genus Trametes is preferable, and a laccase derived from Trametes hirsuta is particularly preferable, from the viewpoint of further increasing the strength enhancing effect of tofu.

The use amount of the laccase is not particularly limited, but the use amount of the laccase per 1 g of soybean protein is, for example, 5 U or more. From the viewpoint of further increasing the strength enhancing effect of tofu, the use amount of the laccase per 1 g of soybean protein is preferably 10 U or more, 20 U or more, or 30 U or more, more preferably 50 U or more, further preferably 60 U or more, even more preferably 120 U or more, further more preferably 180 U or more, and particularly preferably 240 U or more. The upper limit of the use amount range of the laccase is not particularly limited, but the use amount of the laccase per 1 g of soybean protein is, for example, 400 U or less, 300 U or less, or 250 U or less.

As for the laccase activity, when 0.1 ml of a laccase liquid is added to 3 ml of a 1.0 mg/ml solution of 2,2'-Azino-di-[3-ethylbenzthiazoline sulfonate] (ABTS) as a substrate at 25°C, the amount of the laccase that increases the absorbance at 405 nm by 1.0 OD per minute is defined as 1 unit.

In the crosslinking step, a soy milk composition containing soy milk and a laccase is prepared by mixing a laccase and soy milk (typically, adding a laccase to soy milk), and the soy milk composition is maintained in a heated state, whereby crosslinking of soybean protein by the laccase is allowed to proceed, whereby tofu can be obtained.

The temperature of the soy milk at the time of preparing the soy milk composition is not particularly limited, and the soy milk may be in a non-heated state or in a heated state, but is preferably in a heated state. The temperature when the soy milk is in a heated state is not particularly limited, but is preferably a temperature for allowing crosslinking described below to proceed.

The temperature at which the soy milk composition is provided for allowing crosslinking to proceed can be appropriately determined depending on the optimum temperature of the laccase and the like, and is, for example, 50 to 70°C, preferably 52 to 60°C, and more preferably 54 to 57°C.

The time for crosslinking is not particularly limited, and is, for example, 10 minutes or longer, 20 minutes or longer, or 30 minutes or longer, and preferably 1 hour or longer, although it depends on the scale of the soy milk composition and the like. The upper limit of the time range for crosslinking is not particularly limited, and is, for example, 24 hours or shorter, 12 hours or shorter, 8 hours or shorter, or 4 hours or shorter.

The present invention may further include a coagulating step of causing a coagulant to act in addition to the crosslinking step. The timing of performing the coagulating step is not particularly limited, and thus the order of the coagulating step and the crosslinking step is arbitrary. For example, the coagulating step can be performed simultaneously with the crosslinking step, and specifically, a coagulant can act on soy milk together with the laccase. In this case, the order and timing of adding the laccase and the coagulant to the soy milk are not particularly limited, but it is preferable to add an enzyme and a coagulant substantially simultaneously (for example, there are mentioned a method in which an enzyme is added and mixed, and a coagulant is added and mixed immediately thereafter; a method in which an enzyme composition containing an enzyme and a coagulant is added to soy milk and mixed; and the like) so that the crosslinking reaction by the laccase and the coagulation reaction by the coagulant proceed simultaneously. On the other hand, the coagulating step can also be performed after the crosslinking step, and in this case, specifically, a coagulant can be added after adding a laccase to soy milk and subjecting the mixture to the above-described temperature and time to terminate the crosslinking step.

When the present invention includes the coagulating step, the coagulant that can be used in the coagulating step is not particularly limited, and examples thereof include coagulants generally used in production of tofu. Specifically, examples of the coagulant include a salt coagulant and an acid coagulant. Examples of the salt coagulant include magnesium chloride, magnesium sulfate, calcium sulfate, and calcium chloride, and examples of the acid coagulant include glucono delta-lactone.

These coagulants may be used singly or in combination of a plurality of kinds thereof. Among these, from the viewpoint of further increasing the strength enhancing effect of tofu, the coagulant is preferably a salt coagulant and more preferably magnesium chloride.

The use amount of the coagulant is not particularly limited, and is, for example, 0.1 wt% or more, 0.3 wt% or more, or 0.5 wt% or more. From the viewpoint of further increasing the strength enhancing effect of tofu, the use amount of the coagulant is preferably 0.75 wt% or more and more preferably 0.9 wt% or more or 1 wt% or more. The upper limit of the use amount range of the coagulant is not particularly limited, and is, for example, 4 wt% or less, 3 wt% or less, or 2 wt% or less. In particular, when a boiling step is included, the upper limit of the use amount range of the coagulant is preferably 1.5 wt% or less and more preferably 1.2 wt or less or 1 wt% or less, from the viewpoint of further increasing the strength enhancing effect of tofu. When the boiling step is included, the strength enhancing effect can be effectively obtained even without using a large amount of the coagulant, and thus by suppressing the amount of the coagulant, the taste (particularly bitter taste) derived from the coagulant can also be suppressed. The use amount of these coagulants refers to the final concentration in a soy milk composition containing soy milk, a laccase, and a coagulant to be subjected to the crosslinking step.

The reaction conditions in the coagulating step may vary depending on the coagulant, but general conditions used when tofu is produced can be used. For example, the coagulating reaction can be allowed to proceed by subjecting the coagulating reaction to the same temperature and time as those shown in the crosslinking step.

In the present invention, from the viewpoint of further dramatically increasing the strength enhancing effect of tofu, it is preferable to further include a boiling step after the crosslinking step or after the crosslinking step and the coagulating step when the coagulating step is included.

In the boiling step, the obtained tofu is subjected to boiling. Specifically, the obtained tofu can be boiled in boiling water. More specifically, the obtained tofu is put in a container filled with water and sealed, and the whole container can be boiled in boiling water.

The boiling temperature is, the boiling time is not particularly limited, and is, for example, 1 to 30 minutes, preferably 1 to 10 minutes, and more preferably 3 to 8 minutes.

Examples of the form of tofu to be obtained in the present invention include firm tofu, silken tofu, and packed tofu. Since the production method of the present invention can enhance the strength of tofu, even in the form of silken tofu or packed tofu, the strength of tofu can be effectively enhanced.

### EXAMPLES

Hereinafter, the present invention will be specifically described by means of Examples; however, the present invention is not to be construed as being limited to the following Examples.

### (1) Laccase Activity Value Measurement Method

In the following Test Examples, the enzyme activity of the laccase was measured by the method described below using 2,2'-Azino-di-[3-ethylbenzthiazoline sulfonate] (ABTS, manufactured by Boehringer Mannheim) as a substrate.

ABTS was dissolved in a 25 mM citrate buffer solution (pH 3.2) at a concentration of 1.0 mg/ml to prepare a substrate solution. In a cuvette, 3.0 ml of this substrate solution was placed and preheated at 25°C, a 0.1 ml enzyme liquid was then added, stirred, and incubated at 25°C, and the absorbance at 405 nm after 1 minute and 3 minutes was measured. The amount of the enzyme that increased the absorbance at 405 nm by 1.0 OD per minute under this condition was defined as 1 unit (U).

### (2) Materials

Soy milk: "Organic Soy Milk" manufactured by Meiraku Group, soybean protein concentration: 5 g/100 mL, soybean solid content: 10 wt%
Laccase: Laccase derived from Trametes hirsuta

### [Test Example 1]

A soy milk composition was prepared by heating 20 mL of soy milk at 55°C for 5 minutes, adding a laccase aqueous solution so as to be 50 U per 1 g of soybean protein in the soy milk, mixing the mixture for 5 seconds, adding 0.6 mL (final concentration: 1.0 wt%) of a 10 wt% magnesium chloride aqueous solution, and mixing the mixture for 5 seconds, tofu was prepared by heating the soy milk composition at 55°C for the time shown in "Soy milk composition reaction time" in Table 1 (by simultaneously performing the crosslinking step and the coagulating step), and the obtained tofu was cooled to 4°C (Examples 1-1 to 1-4). Tofu (Comparative Examples 1-1 to 1-4) was prepared in the same manner except that a laccase was not added.

**[Table 1]**

| | Presence or absence of laccase treatment | Laccase use amount (U/1 g soybean protein) | Coagulant use amount (wt%) | Soy milk composition reaction time (hour) | Presence or absence of boiling step |
|---|---|---|---|---|---|
| Example 1-1 | Present | 50 | 1.0 | 0.5 | Absent |
| Example 1-2 | | | | 1 | |
| Example 1-3 | | | | 2 | |
| Example 1-4 | | | | 4 | |
| Comparative Example 1-1 | Absent | 0 | 1.0 | 0.5 | Absent |
| Comparative Example 1-2 | | | | 1 | |
| Comparative Example 1-3 | | | | 2 | |
| Comparative Example 1-4 | | | | 4 | |

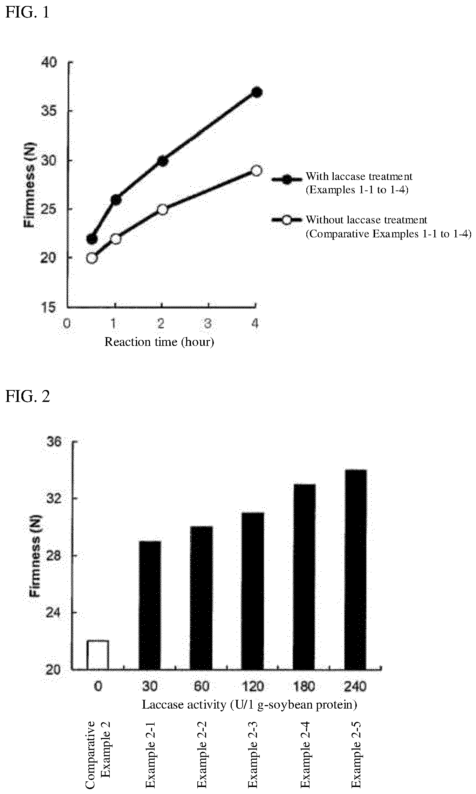

The strength (Firmness (N); specifically, compressive strength) of the obtained tofu was measured using a rheometer (COMPAC-100II SUN SCIENTIFIC CO., LTD.). The measurement conditions were as follows: Mode: 20, adapter: No. 13, repetition: 1 time, and pushing distance: 5 mm. Results are shown in Fig. 1.

As shown in Fig. 1, it was shown that the strength of the tofu of each of Examples 1-1 to 1-4 prepared by performing the laccase treatment was more enhanced than that of the tofu of each of Comparative Examples 1-1 to 1-4 prepared by only performing a coagulant treatment without performing the laccase treatment.

### [Test Example 2]

Tofu (Comparative Example 2 and Examples 2-1 to 2-5) was prepared in the same manner as in Test Example 1, except that the use amount of the laccase was set to the amount shown in Table 2 and the heating time of the soy milk composition in the crosslinking step and the coagulating step was set to 1 hour.

**[Table 2]**

| | Presence or absence of laccase treatment | Laccase use amount (U/1 g soybean protein) | Coagulant use amount (wt%) | Soy milk composition reaction time (hour) | Presence or absence of boiling step |
|---|---|---|---|---|---|
| Comparative Example 2 | Absent | 0 | 1.0 | 1 | Absent |
| Example 2-1 | Present | 30 | 1.0 | 1 | Absent |
| Example 2-2 | | 60 | | | |
| Example 2-3 | | 120 | | | |
| Example 2-4 | | 180 | | | |
| Example 2-5 | | 240 | | | |

The strength (Firmness (N)) of the obtained tofu was measured in the same manner as in Test Example 1. Results are shown in Fig. 2.

As shown in Fig. 2, in the tofu of each of Examples 2-1 to 2-5 in which the laccase was used in a use amount of 30 U or more per 1 g of soybean protein, the strength thereof was clearly enhanced as compared with the tofu of Comparative Example 2 in which the laccase treatment was not performed. Specifically, when the strength of the tofu of Comparative Example 2 was regarded as "1", the ratios of the strengths of the tofu of each of Examples 2-1 to 2-5 were 1.32 times, 1.36 times, 1.41 times, 1.50 times, and 1.55 times, respectively.

### [Test Example 3]

Tofu (Examples 3-1 to 3-5) was prepared in the same manner as in Test Example 1, except that the use amount of the coagulant (magnesium chloride) was set to the amount (final concentration) shown in Table 3 and the heating time of the soy milk composition in the crosslinking step and the coagulating step was set to 1 hour, and tofu (Comparative Examples 3-1 to 3-5) was prepared in the same manner as in Test Example 1, except that the above-described points were set and the laccase treatment was not performed.

**[Table 3]**

| | Presence or absence of laccase treatment | Laccase use amount (U/1 g soybean protein) | Coagulant use amount (wt%) | Soy milk composition reaction time (hour) | Presence or absence of boiling step |
|---|---|---|---|---|---|
| Example 3-1 | Present | 50 | 0.5 | 1 | Absent |
| Example 3-2 | | | 0.75 | | |
| Example 3-3 | | | 1.0 | | |
| Example 3-4 | | | 1.5 | | |
| Example 3-5 | | | 2.0 | | |
| Comparative Example 3-1 | Absent | 0 | 0.5 | 1 | Absent |
| Comparative Example 3-2 | | | 0.75 | | |
| Comparative Example 3-3 | | | 1.0 | | |
| Comparative Example 3-4 | | | 1.5 | | |
| Comparative Example 3-5 | | | 2.0 | | |

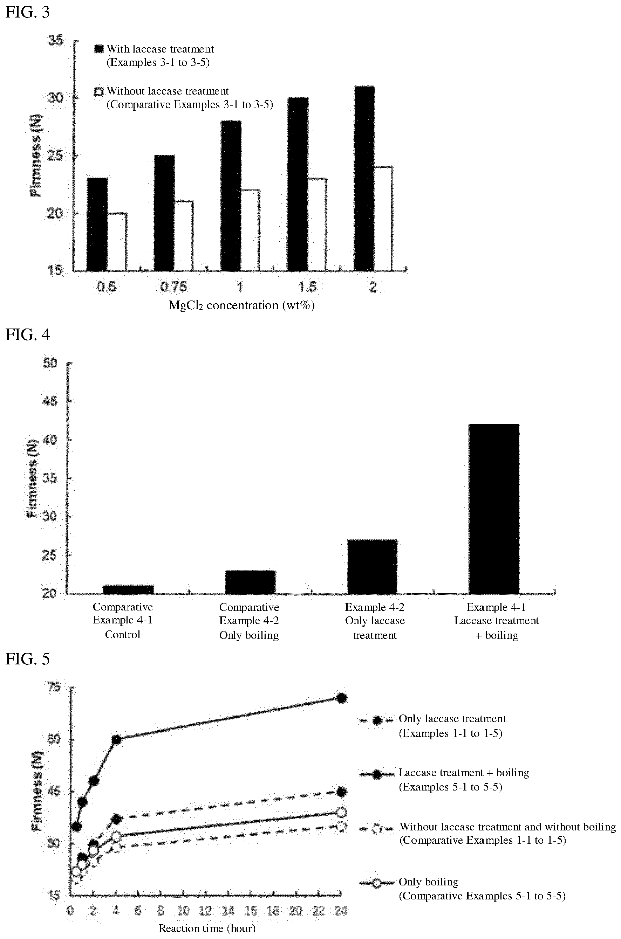

The strength (Firmness (N)) of the obtained tofu was measured in the same manner as in Test Example 1. Results are shown in Fig. 3. As shown in Fig. 3, the strength of the tofu (Examples 3-1 to 3-5) was enhanced according to the use amount of the coagulant.

When the strength of the tofu of each of Comparative Examples 3-1 to 3-5 in which the laccase treatment was not performed was regarded as "1", the ratios of the strengths of the tofu of each of Examples 3-1 to 3-5 in which the laccase treatment was performed were 1.15 times (in the case of 0.5 wt% MgCh), 1.19 times (in the case of 0.75 wt% MgCl₂), 1.27 times (in the case of 1 wt% MgCl₂), 1.30 times (in the case of 1.5 wt% MgCh), and 1.29 times (in the case of 2 wt% MgCh), respectively. Therefore, it was found that when the use amount of the coagulant was 1 to 2 wt%, the effect of enhancing the strength of the tofu was particularly high.

### [Test Example 4]

Tofu (Example 4-1) was prepared in the same manner as in Test Example 1, except that the heating time of the soy milk composition in the crosslinking step and the coagulating step was set to 1 hour and boiling was performed at 100°C for 5 minutes (boiling step was performed) after the crosslinking step and the coagulating step. In the boiling step, the prepared tofu was put in a container filled with water and sealed, and the whole container was boiled in boiling water. For comparison with the tofu of Example 4-1, tofu prepared without performing the laccase treatment and the boiling (Comparative Example 4-1), tofu prepared by performing only boiling without performing the laccase treatment (Comparative Example 4-2), and tofu prepared by performing the laccase treatment without performing boiling (Example 4-2) were also prepared. The tofu prepared for comparison was prepared under the same conditions as those of the tofu of Example 4-1, except that the above-described conditions were set for the presence or absence of the laccase treatment and boiling as shown in Table 4.

**[Table 4]**

| | Presence or absence of laccase treatment | Laccase use amount (U/1 g soybean protein) | Coagulant use amount (wt%) | Soy milk composition reaction time (hour) | Presence or absence of boiling step |
|---|---|---|---|---|---|
| Example 4-1 Laccase treatment + boiling | Present | 50 | 1.0 | 1 | Present |
| Example 4-2 Only laccase treatment | | | | | Absent |
| Comparative Example 4-1 Control | Absent | 0 | 1.0 | 1 | Absent |
| Comparative Example 4-2 Only boiling | | | | | Present |

The strength (Firmness (N)) of the obtained tofu was measured in the same manner as in Test Example 1. Results are shown in Fig. 4.

As shown in Fig. 4, in addition to the strength being enhanced in the tofu obtained by only the laccase treatment (Example 4-2) as compared with the tofu of control (Comparative Example 4-1), the strength of the tofu obtained by combining the laccase treatment and boiling (Example 4-1) was dramatically enhanced to be 1.6 times as compared with the tofu obtained by only the laccase treatment (Example 4-2) (2 times as compared with Comparative Example 4-1). In view of the fact that the strength of the tofu obtained by only boiling (Comparative Example 4-2) was enhanced only by 1.09 times as compared with the tofu of control (Comparative Example 4-1), it can be said that the strength enhancing effect observed in the tofu obtained by combining the laccase treatment and boiling (Example 4-1) synergistically exhibited the strength enhancing effect by the laccase treatment and the strength enhancing effect by boiling.

### [Test Example 5]

Tofu (Examples 5-1 to 5-5 and Comparative Examples 5-1 to 5-5) was prepared in the same manner as in Test Example 1, except that the soy milk composition was heated for the time shown in "Soy milk composition reaction time" in Table 5 (crosslinking step) in the crosslinking step and boiling was performed at 100°C for 5 minutes (boiling step was performed) after the crosslinking step.

**[Table 5]**

| | Presence or absence of laccase treatment | Laccase use amount (U/1 g soybean protein) | Coagulant use amount (wt%) | Soy milk composition reaction time (hour) | Presence or absence of boiling step |
|---|---|---|---|---|---|
| Example 5-1 | Present | 50 | 1.0 | 0.5 | Present |
| Example 5-2 | | | | 1 | |
| Example 5-3 | | | | 2 | |
| Example 5-4 | | | | 4 | |
| Example 5-5 | | | | 24 | |
| Comparative Example 5-1 | Absent | 0 | 1.0 | 0.5 | Present |
| Comparative Example 5-2 | | | | 1 | |
| Comparative Example 5-3 | | | | 2 | |
| Comparative Example 5-4 | | | | 4 | |
| Comparative Example 5-5 | | | | 24 | |

The strength (Firmness (N)) of the obtained tofu was measured in the same manner as in Test Example 1. Results are shown in Fig. 5. Fig. 5 also shows the results of Examples 1-1 to 1-4 and Comparative Examples 1-1 to 1-4 of Test Example 1, and the results of the tofu obtained when the reaction time was 24 hours (described as Example 1-5 and Comparative Example 1-5) in these Examples and Comparative Examples.

As shown in Fig. 5, in the tofu obtained by performing boiling (Examples 5-1 to 5-5 and Comparative Examples 5-1 to 5-5), a remarkable strength enhancing effect was observed in the tofu obtained by performing the laccase treatment (Examples 5-1 to 5-5). The strength of the tofu was enhanced by performing the laccase treatment similarly to the tofu in which boiling was not performed (Examples 1-1 to 1-5 and Comparative Examples 1-1 to 1-5), but the strength enhancing effect by the laccase treatment was remarkably exhibited when boiling was performed. For example, when the reaction time for the crosslinking step was 1 hour, the strength of the tofu without boiling in which the laccase treatment was performed (Example 1-2) was enhanced by 1.2 times as compared with the tofu without boiling in which the laccase treatment was not performed (Comparative Example 1-2); on the other hand, the strength of the tofu with boiling in which the laccase treatment was performed (Example 5-2) was enhanced by 1.75 times as compared with the tofu with boiling in which the laccase treatment was not performed (Comparative Example 5-2).

The ratio of the strength of the tofu in which boiling was performed (Examples 5-1 to 5-5 and Comparative Examples 5-1 to 5-5) to the strength of the tofu in which boiling was not performed (Examples 1-1 to 1-5 and Comparative Examples 1-1 to 1-5) did not change over the reaction time of 0.5 to 24 hours, and specifically, in the case without the laccase treatment, the strength enhancing effect by the boiling treatment (Comparative Examples 5-1 to 5-5) was maintained 1.1 times the strength enhancing effect in the case without boiling (Comparative Examples 1-1 to 1-5) over the reaction time of 0.5 to 24 hours, and in the case with the laccase treatment, the strength enhancing effect by the boiling treatment (Examples 5-1 to 5-5) was maintained 1.6 times the strength enhancing effect in the case without boiling (Examples 1-1 to 1-5) over the reaction time of 0.5 to 24 hours.

### [Test Example 6]

Tofu (Comparative Example 6 and Examples 6-1 to 6-5) was prepared in the same manner as in Test Example 1, except that the use amount of the laccase was set to the amount shown in Table 6, the heating time of the soy milk composition in the crosslinking step and the coagulating step was set to 1 hour, and boiling was performed at 100°C for 5 minutes (boiling step was performed) after the crosslinking step.

**[Table 6]**

| | Presence or absence of laccase treatment | Laccase use amount (U/1 g soybean protein) | Coagulant use amount (wt%) | Soy milk composition reaction time (hour) | Presence or absence of boiling step |
|---|---|---|---|---|---|
| Comparative Example 6 | Absent | 0 | 1.0 | 1 | Present |
| Example 6-1 | Present | 30 | 1.0 | 1 | Present |
| Example 6-2 | | 60 | | | |
| Example 6-3 | | 120 | | | |
| Example 6-4 | | 180 | | | |
| Example 6-5 | | 240 | | | |

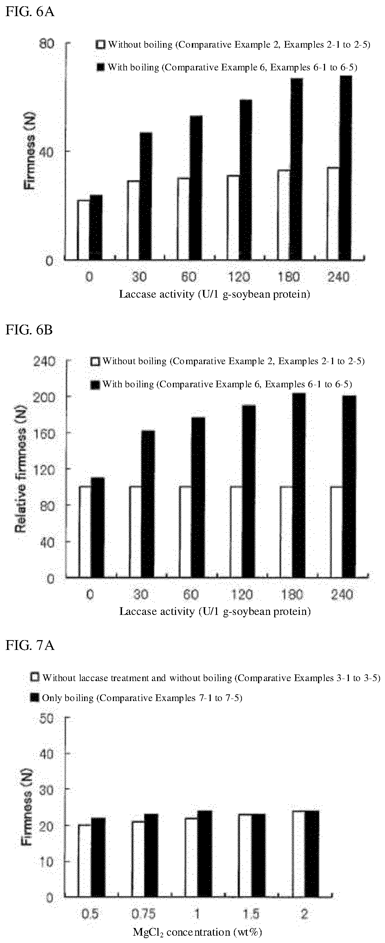

The strength (Firmness (N)) of the obtained tofu was measured in the same manner as in Test Example 1. Results are shown in Fig. 6A. Fig. 6A also shows the results of Comparative Example 2 and Examples 2-1 to 2-5 of Test Example 2.

As shown in Fig. 6A, in the tofu of each of Examples 6-1 to 6-5 in which the laccase was used in a use amount of 30 U or more per 1 g of soybean protein, the strength thereof was remarkably enhanced as compared with the tofu of Comparative Example 6 in which the laccase treatment was not performed. Specifically, when the strength of the tofu of Comparative Example 6 was regarded as "1", the ratios of the strengths of each of the tofu of Examples 6-1 to 6-5 were 1.96 times, 2.21 times, 2.46 times, 2.79 times, and 2.83 times, respectively.

Fig. 6B shows the relative strength (Relative firmness (N)) of the tofu of each of Comparative Example 6 and Examples 6-1 to 6-5 when the strength of the tofu without boiling (Comparative Example 2 and Examples 2-1 to 2-5) is regarded as "100" in each laccase use amount. As shown in Fig. 6B, the relative strength of Examples 6-1 to 6-5 was remarkably enhanced as compared with the relative strength of Comparative Example 6, and it was shown that the strength enhancing effect of tofu by boiling can be further improved by performing the laccase treatment. As shown in Examples 6-1 to 6-4, since the relative strength was enhanced as the use amount of the laccase was increased up to 180 U per 1 g of soybean protein, it was shown that the effect of increasing the tofu strength associated with the use amount of the laccase was further improved by boiling.

### [Test Example 7]

Tofu (Examples 7-1 to 7-5) was prepared in the same manner as in Test Example 1, except that the use amount of the coagulant (magnesium chloride) was set to the amount (final concentration) shown in Table 7, the heating time of the soy milk composition in the crosslinking step and the coagulating step was set to 1 hour, and boiling was performed at 100°C for 5 minutes (boiling step) after the crosslinking step and the coagulating step, and tofu (Comparative Examples 7-1 to 7-5) was prepared in the same manner as in Test Example 1, except that the above-described points were set and the laccase treatment was not performed.

**[Table 7]**

| | Presence or absence of laccase treatment | Laccase use amount (U/1 g soybean protein) | Coagulant use amount (wt%) | Soy milk composition reaction time (hour) | Presence or absence of boiling step |
|---|---|---|---|---|---|
| Example 7-1 | Present | 50 | 0.5 | 1 | Present |
| Example 7-2 | | | 0.75 | | |
| Example 7-3 | | | 1.0 | | |
| Example 7-4 | | | 1.5 | | |
| Example 7-5 | | | 2.0 | | |
| Comparative Example 7-1 | Absent | 0 | 0.5 | 1 | Present |
| Comparative Example 7-2 | | | 0.75 | | |
| Comparative Example 7-3 | | | 1.0 | | |
| Comparative Example 7-4 | | | 1.5 | | |
| Comparative Example 7-5 | | | 2.0 | | |

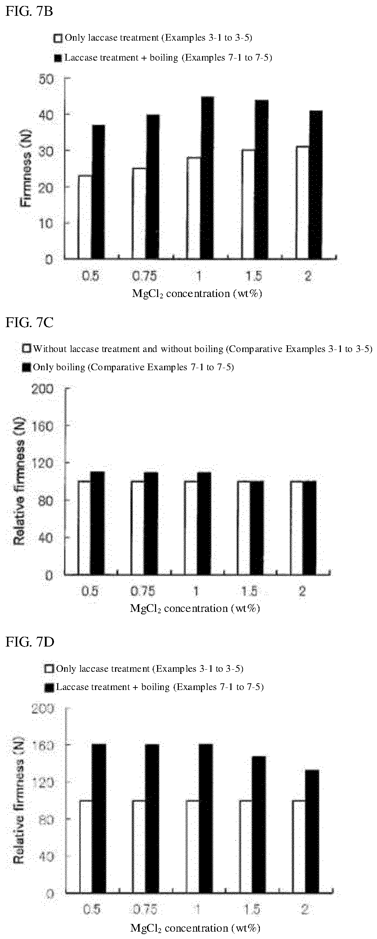

The strength (Firmness (N)) of the obtained tofu was measured in the same manner as in Test Example 1. Results are shown in Figs. 7A and 7B. Fig. 7A also shows the results of Comparative Examples 3-1 to 3-5 of Test Example 3, and Fig. 7B also shows the results of Examples 3-1 to 3-5 of Test Example 3. Figs. 7C and 7D show the relative strength (Relative firmness (N)) of the tofu of each of Comparative Examples 7-1 to 7-5 and Examples 7-1 to 7-5 when the strength of the tofu without boiling (Comparative Examples 3-1 to 3-5 and Examples 3-1 to 3-5) is regarded as "100" in each coagulant use amount.

As shown in Figs. 7A and 7C, in the tofu without the laccase treatment, when the use amount of the coagulant was 0.5 wt%, 0.75 wt%, and 1.0 wt%, the strength was slightly enhanced by boiling (Comparative Examples 7-1 to 7-3), but when the use amount of the coagulant was 1.5 wt% and 2.0 wt%, the strength enhancing effect was not observed even by boiling (Comparative Examples 7-4 to 7-5). On the other hand, as shown in Figs. 7B and 7D, in the tofu with the laccase treatment, a remarkable strength enhancing effect was observed by boiling not only when the use amount of the coagulant was 0.5 wt%, 0.75 wt%, and 1.0 wt%, but also when the use amount of the coagulant was 1.5 wt% and 2.0 wt% (Examples 7-1 to 7-5).

## Claims

1. A tofu production method comprising a crosslinking step of causing a laccase to act on soy milk.

2. The tofu production method according to claim 1, further comprising a boiling step after the crosslinking step.

3. The tofu production method according to claim 1 or 2, further comprising a coagulating step of causing a coagulant to act.

4. The tofu production method according to any one of claims 1 to 3, wherein the laccase is used in an amount of 5 U or more per 1 g of soybean protein.

5. The tofu production method according to any one of claims 1 to 4, wherein the laccase is used in an amount of 30 U or more per 1 g of soybean protein.

6. The tofu production method according to any one of claims 1 to 5, wherein the laccase is derived from Trametes hirsuta.

7. The tofu production method according to any one of claims 1 to 6, wherein the crosslinking step is performed at 50 to 70°C.

8. The tofu production method according to any one of claims 3 to 7, wherein the coagulant is used at a concentration of 0.1 wt% or more.

9. The tofu production method according to any one of claims 3 to 8, wherein the coagulant is used at a concentration of 0.5 wt% or more.

10. The tofu production method according to any one of claims 3 to 9, wherein the coagulant is magnesium chloride.
